# EUROPEAN PATENT SPECIFICATION

(11) **EP 1 581 258 B1**
(45) Date of publication and mention of the grant of the patent: **31.08.2016**
(21) Application number: 03781126.2
(22) Date of filing: 12.12.2003
(51) Int. Cl.: A61K 9/14, A61K 9/00, A61K 47/14, A61K 31/365, A61P 33/14, A61P 33/10, A61K 31/4184, A61K 31/425, A61K 31/495, A61K 31/7048, A61K 45/06

(54) **PHARMACEUTICAL FORMULATION AND A METHOD OF MAKING SAME**
PHARMAZEUTISCHE FORMULIERUNG UND HERSTELLUNGSVERFAHREN DAFÜR
FORMULATION PHARMACEUTIQUE ET PROCEDE DE REALISATION ASSOCIE

(30) Priority: 12.12.2002 NZ 52312802
(43) Date of publication of application: 05.10.2005
(73) Proprietor: Merial Limited, Harlow, Essex CM19 5QA (GB)
(72) Inventor: RAZZAK, Majid Hameed Abdul, Albany, 1309 Auckland (NZ); GAMAGE, Ranjith Srilal, Hillsborough, 1004 Auckland (NZ); RAO, Bantupalli, Mt Wellington, 1006 Auckland (NZ)
(74) Representative: D Young & Co LLP
(86) International application number: PCT/NZ2003/000272
(87) International publication number: WO 2004/052400

(56) References cited:
- WO-A-01/60409
- WO-A1-00/74489
- WO-A1-02/09764
- WO-A1-98/06407
- US-A- 4 678 774
- PATENT ABSTRACTS OF JAPAN & JP 05 085 902 A (NIPPON KAYAKU CO LTD) 06 April 1993

## Description

### FIELD OF THE INVENTION

This invention relates to pharmaceutical formulations, their preparation and their use in veterinary applications.

More specifically, the present description relates to methods of making multi-active formulations which are capable of stably integrating multiple actives.

### BACKGROUND

It is often desirable to administer a combination of actives. A mixture may be required for effective management of a condition, or the combination may give an advantageous result over the administration of a single active.

In most human health situations the administration of multiple formulations is acceptable. However, in circumstances where mass administration of a combination of actives is required, it is advantageous if the combination can be administered in the same formulation. This is especially the case where inoculating a large proportion of the population or in the veterinary applications where a herd of animals may require treatment.

In some cases, the actives to be co-adnainistered will have overlapping formulation requirements and should to co-exist stably in the formulation.

However, in many cases, the actives to be co-administered will have conflicting formulation requirements or may actively degrade each other. By way of example, levamisole is soluble in water-based formulations and requires an acidic pH for stability. By contrast avermectins are substantially insoluble in water. If formulated in water based formulation avermectins require a neutral pH for stability. In the acid pH required by levamisole the avermectins will degrade.

In addition, to the need for finding a formulation in which each of the actives can stably integrate, the formulation must be practical to use. That is, it must be able to be stored for say up to a year without significant physical or chemical changes taking place. The relative proportions of the components should not change significantly over time. In addition, while some sedimentation is to be expected, the sediment should be easily re-dispersed on shaking. The formulation should not cause excessive side effects in the animal. The formulation should be relatively easy to administer and it is of suitable flowability to allow delivery by injection and drench.

Various attempts have been made to produce effective combined active formulations with limited success.

One such attempt is set out in New Zealand Patent Application No. 336139, which involves a storage stable veterinary composition comprising a levamisole containing aqueous phase and an anthelmintic containing organic phase wherein the composition having at least most of the first active in the organic phase is emulsified in the second liquid phase which includes the second active agent when present. However, this suspo-emulsion method, as it is known, tends to result in a formulation that is highly viscous and flows poorly. As such, it is difficult to administer.

WO 02/09764 A1 discloses a composition enabling the incorporation of both lipophilic and hydrophilic drugs. WO 02/09764 A1 does not disclose a sorbing medium.

Accordingly there is a need for an improved formulation capable of integrating a number of actives together with a method of manufacturing the same.

### OBJECT

It is the object of the present description to provide an improved multi-active formulation and a method of making the same or one that will at least provide the public with a useful choice.

### STATEMENT OF THE INVENTION

The present invention provides a method of manufacturing a pharmaceutical formulation comprising the steps:
a) dissolving an active in a solvent, wherein the active is a lipophilic active and wherein the lipophilic active is selected from the group consisting of avermectins and the milbemycins;
b) sorbing the solvent containing active composition on to a sorbing medium wherein the sorbing medium is hydrophobic fumed silica; and then
c) dispersing the sorbing medium loaded with the solvent composition in a liquid comprising one or more actives dissolved or suspended therein wherein the active is a vitamin, mineral, anthelmintics or antigen and wherein the liquid is an aqueous vehicle.

In another aspect, the present invention provides a pharmaceutical formulation capable of dilution and capable (with or without dilution) of being administered to an animal, said formulation comprising:
a) an active dissolved in a suitable solvent to form a solution and sorbed on to a sorbing medium, wherein the active is a lipophilic active and wherein the lipophilic active is selected from the group consisting of avermectins and the milbemycins, and wherein the sorbing medium is hydrophobic fumed silica; and
b) a liquid diluent wherein the liquid diluent is an aqueous vehicle comprising one or more actives dissolved or suspended therein wherein the active is a vitamin, mineral, anthelmintics or antigen.

In a further aspect, the present invention provide the use of a formulation according to the present invention in the manufacture of a medicament formulated for administration to an animal.

The present invention provides, in a further aspect, a formulation according to the present invention for use in a medicament formulated for administration to an animal.

In one aspect the description comprises a method of manufacturing a pharmaceutical formulation comprising the steps:
a) Dissolving an active in a solvent;
b) Sorbing the solvent containing active composition on to a sorbing medium; and then
c) Dispersing the sorbing medium loaded with the solvent composition in a liquid.

Preferably the active is a lipophilic active.

More preferably the lipophilic active is selected from the group including the avermectins and the milbemycins.

Preferably the liquid is an aqueous vehicle.

Preferably the solvent is selected from oils and organic solvents.

More preferably the solvent is selected from medium chain mono-/di-glycerides (Capmul MCM) in the range of 0.1-10% preferably between 2-4%.

Preferably the liquid optionally includes one or more medicaments selected from the group including vitamins, minerals, anthelmintics or antigens.

Preferably the minerals are selected from selenium salts, cobalt slats, copper salts, zinc salts and iodine salts and their chelates.

More preferably the minerals are sodium selenate and cobalt EDTA.

Preferably the anthelmintics are selected from the group including thiazole derivatives such as a levamisole salt and benzimidazole derivates such as albendazole, oxfendazole, fenbendazole, mebendazole and acylated quinoline such as praziquantel and benzenesulphamide such as clorsulon and closantel.

More preferably the sorbing medium is selected from magnesium aluminometasilicate, cellulose, microcrystalline cellulose, diatomaceous earth, montmorillonite, betonite, titanium dioxide, amorphous silicon dioxide, colloidal silicon dioxide, calcium carbonate, talc (SiO2+MgO), attapulgite (silicon, aluminium and iron oxides clay), kaolin (aluminium silicate) preferably colloidal silicon dioxide (Aerosil) more preferably Aerosil R972.

Preferably the liquid optionally comprises further excipients including but not limited to preservatives, suspending agents, buffering agents, antifoaming agents and the like.

In a further related aspect the description comprises a pharmaceutical formulation capable of dilution and capable (with or without dilution) of being administered to an animal, said formulation comprising:
a) an active dissolved in a suitable solvent to form a solution and sorbed on to a sorbing medium; and
b) Preferably including an liquid diluent.

Preferably the active is a lipophilic active.

Preferably wherein the active is selected from the group including the avermectins and the milbemycins.

Preferably the solvent is selected from oils and organic solvents.

More preferably the solvent is medium chain mono-/di-glycerides (Capmul MCM).

Preferably the liquid diluent is an aqueous vehicle.

More preferably the liquid diluent optionally includes one or more additional medicaments selected from the group including vitamins, minerals, anthelmintics or antigens.

Preferably the medicament is either soluble and/or dispersible in the liquid.

Preferably the minerals are selected from wherein the minerals are selected from selenium salts, cobalt salts, copper salts, zinc salts and iodine salts and their chelates.

More preferably the minerals are selected from sodium selenate and cobalt EDTA.

Preferably the anthelmintics are selected from the group including thiazole derivatives such as a levamisole salt and benzimidazole derivates such as albendazole, oxfendazole, fenbendazole, mebendazole and acylated quinoline such as praziquantel, and benzenesulphonamide such as a clorsulon and closantel.

Preferably the formulation additionally includes preservatives, suspending agents, buffering agents, antifoaming agents and the like.

In a further related aspect the description relates to a method of medical treatment comprising administering a formulation as described in the preceding statements to an animal.
"Solvent" means an oil or water immiscible liquid having good solubility for the active and being able to sorb onto the silica or other sorbing medium to physically separate the first active from the liquid.
"Sorb and sorbing" is used to include the absorption and adsorption processes.
"Active" includes compounds which provide a health advantage or benefit to an animal to which the formulations referred to in the above paragraph are administered. Active includes vitamins, minerals, anthelmintics, antigens and the like.
"Liquid" referred to in the preceding paragraphs comprises a liquid carrier optionally including one or more actives dissolved or suspended therein. The liquid may be water or oil based liquid.

In preferred examples the liquid will be an aqueous vehicle, that is it will be water based and will include a water soluble active dissolved therein. It is envisaged however that the liquid may also include a further active suspended therein.

The liquid may also include excipients including defoaming agents and buffering agents.

The method requires the dissolving of a first active in a suitable solvent. This solution is sorbed onto a sorbing medium such as silica dioxide (e.g. Aerosil R972). This active loaded medium is then dispersed in a liquid.

Suitable sorbing mediums include, by way of example, magnesium aluminometasilicate, cellulose, microcrystalline cellulose, diatomaceous earth, montmorillonite, bentonite, titanium dioxide, amorphous silicon dioxide, calcium carbonate, talc (SiO2+MgO), attapulgite (silicon, aluminium and iron oxides clay) and kaolin (aluminum silicate).

All these exhibit large specific surface area and have high adsorption capacity and can be used to sorb the solvent-containing drug.

Hydrophobic fumed silica is also suitable for this application, as it tend to produce less viscous product when mixed with oil. The oil adsorption capacity is very good and the resulting mix is free flowing and can be dispersed easily with the bulk of the aqueous formulation.

The liquid in which the sorbing medium is to be dispersed may itself be a composition including an active. The liquid may comprise a water or oily based liquid and may include appropriate actives, such as levamisole, sodium selenate and the like. In addition, to the active, the liquid may include suspension aids/viscosity inducing agents such as sodium carboxy methylcellulose, guar gum, karaya gum or xanthan gum. In addition, preservatives, such as methylparaben, propylparaben and their salts may be used to prevent degradation of the formulation.

A defoamer, such as a silicon emulsion can be used as a lubricant.

Further, suspension aids may be incorporated within the formulation.

In addition it is envisaged the formulation method may incorporate other actives including vitamins, minerals and the like as actives. The same actives may also be dissolved in the formulation and sorbed onto the silica or other sorbing medium to provide differential release of the active.

Further, it is envisaged additional avermectins or other anthelmintics may be incorporated into the formulation. These additional actives may be incorporated in the suspension or sorbed onto the silica or other sorbing medium to provide for differential release of the active.

The present description relates to a method for making a formulation incorporating multiple actives. The formulation and method are advantageous as they allow the stable integration of actives, which have different formulation requirements, in the same formulation.

The present description is advantageous in that it allows the incorporation in a single formulation of multiple actives, which may be incompatible in that they have different solubility characteristics or requirements or they degrade the other.

It is important when arriving at the combination of actives and solvents to be used that the active and solvent sorbed on to the sorbing medium should be incompatible with the liquid in which the active loaded sorbing medium is dispersed. By way of example if a lipophilic active, such as an avermectin is dissolved in a lipophilic solvent such as Capmul MCM and loaded onto Aerosil, the liquid should be an aqueous based.

An important factor in determining suitable active and solvent combinations for loading onto the Aerosil is the amount of active needed to provide effective treatment. Accordingly active which provide good efficacy at relatively low dose rates are more suitable for sorption onto the Aerosil than are actives which require administration of a relatively large dose for efficacy. The constraint is a physical one, the inclusion of a larger amount of Aerosil or other sorbing medium into a formulation can lead to difficulties in flowability.

### DETAILED DESCRIPTION

The present description provides an improved veterinary composition including two or more actives together with a method of manufacturing the same.

### DEVELOPMENT TRIALS

The following examples numbered 1 to 13, relate to trials to determine an effective formulation. These are given by way of illustration.

The flow time of each example formulation that determined by timing how long it took for a volume of 100 ml to flow through a ford no. 4 cup. A flow time of less than about 20 seconds was desired.

In addition, the chemical and physical stability of the formulation was checked. The formulations were exposed to 55°C for four weeks after which the levels of the actives and then condition of the formulation was checked.

### Example 1

The initial trial included silicon dioxide (brand name Aerosil R972) and polyoxyl 40 hydrogenated castor oil.

The formulation comprising:
1. 3.5g Carboxymethyl cellulose sodium is dissolved in 150ml of water.
2. 500mg Ivermectin is dissolved in 25ml of medium chain mono-/di-glycerides (Capmul MCM.)
3. 900mg Sodium methyl paraben and 100mg sodium propyl paraben are dissolved in 250ml water, wherein 476mg sodium selenate and 6.284g cobalt EDTA are added under stirring.
4. 20g Levamisole HCl is added to the formulation from step 3 under stirring, and 10g polyoxyl 40 hydrogenated caster oil and 500mg defoamer are added under stirring.
5. 11.35g Oxfendazole is dispersed into the formulation resulting from step 4 under stirring.
6. Medium chain mono-/di-glycerides containing ivermectin (the formulation from step 2) is sorbed on 15g silicon dioxide (brand name Aerosil R972) using mortar and pestle.
7. The ivermectin-loaded silicon dioxide (brand name Aerosil R972) is dispersed into the formulation of step 5 under stirring.
8. The formulation of Step 1 is added to that from step 7 under stirring.
9. 10ml of purified water is used to rinse the beaker; this is then added to the formulation from step 8.
10. The pH is adjusted to below 4, preferably 3.84 using 20%w/v citric acid solution.
11. Volume is made up to 500ml using purified water.

The result of this combination is a physically and chemically stable formulation with a flow time through a Ford No. 4 cup of 33 seconds.

### Example 2

In a further trial the silicon dioxide (brand name Aerosil R972) was used alone to provide a formulation which was surfactant free.

The formulation components and method are as in example 1 except:
- At step 4 the defoamer is added but the polyoxyl 40 hydrogenated castor oil is omitted; and
- At step 10 the pH is adjusted to 3.80 instead of 3.84 in example 1

The result of this combination is a physically and chemically stable formulation with a flow time through a Ford No. 4 cup of around 27 seconds.

### Reference example 3

In a further trial the silicon dioxide (brand name Aerosil 200) was used in combination with polyoxyl 40 hydrogenated castor oil.

The formulation components and methods are as example 1 except:
- At step 6 the medium chain mono-/di-glycerides containing ivermectin are sorbed on silicon dioxide (brand name Aerosil 200) instead of silicon dioxide (brand name Aerosil R972), and
- At step 10 the pH is adjusted to 3.76 instead of 3.84 in example 1.

The resulting formulation was chemically stable; however it exhibited a non-homogeneous sedimentation. Accordingly it was decided to discontinue this formulation. The flow time for this formulation was 28 seconds.

### Reference example 4

In a further trial silicon dioxide (brand name Aerosil 200) was used alone to provide a formulation which was surfactant free.

The formulation components and methods are as in example 1, except:
- At step 4 the defoamer is added and the polyoxyl 40 hydrogenated castor oil is omitted, and
- At step 6 medium chain mono-/di-glycerides containing ivermectin are sorbed on to silicon dioxide (brand name Aerosil 200) instead of silicon dioxide (brand name Aerosil R972).
- At step 10 the pH is adjusted to 3.81 instead of 3.84 in example 1.
- The formulation has a flow time of 17 seconds, however, it did not exhibit sufficient physical stability, accordingly it was decided to discontinue this formulation.

### Reference example

In a further trial silicon dioxide (brand name Aerosil 200) and polyoxyl 40 hydrogenated castor oil were used for reconfirmation of the results in example 3.

The formulation components and methods are as example 3 except:
- At step 10 the pH is adjusted to 3.80 instead of 3.76 in example 1.

The resulting formulation was surfactant free and chemically stable. This confirmed the formulation of example 3 was viable. However, it exhibited a flow time of 30 seconds.

### Example 6

It was decided to use the formulation in example 2 as a base but to use 0.5% carboxymethyl cellulose sodium. The formulation volume was increased to 1000ml. The amount of each component was increased accordingly.

The formula in this example uses silicon dioxide (brand name Aerosil R972) alone and 0.5% carboxymethyl cellulose sodium to improve the flow time.

The formulation components and methods are as follows:
1. 5g Carboxymethyl cellulose sodium is dissolved in 300ml of water.
2. 1.072mg Ivermectin is dissolved in 50ml of medium chain mono-/di-glycerides (Capmul MCM.)
3. 1.8mg Sodium methyl paraben and 200mg sodium propyl paraben are dissolved in 500ml water, wherein 958mg sodium selenate and 12.568g cobalt EDTA are added under stirring.
4. 40g Levamisole HCl is added to the formulation from step 3 and 1g defoamer added under stirring.
5. 22.7g Oxfendazole is dispersed into the formulation resulting from step 4 under stirring.
6. Medium chain mono-/di-glycerides containing ivermectin (the formulation from step 2) is sorbed on 30g silicon dioxide (brand name Aerosil R972) using mortar and pestle.
7. The ivermectin-loaded silicon dioxide (brand name Aerosil R972) is dispersed into the formulation of step 5 under stirring.
8. The formulation of Step 1 is added to that from step 7 under stirring.
9. 20ml of purified water is used to rinse the beaker; this is then added to the formulation from step 8.
10. The pH is adjusted to below 4, preferably 3.84 using 20%w/v citric acid solution.
11. Volume is made up to 1L using purified water.

Testing of this formulation revealed a flow rate of 16 seconds through a Ford No. 4 cup suggesting the defoamer had no effect on the flow rate.

### Example 7

In a further trial silicon dioxide (brand name Aerosil R972) was used and 0.5% carboxymethyl cellulose sodium was added for changing stability.

The formulation components and methods are as example 6 except:
- At step 10 the pH is set at 3.79 instead of 3.84 and 30%w/v citric acid solution is used instead of 20%w/v in example 6.
- At step 11 the volume is made up to 1000ml using purified water.

Testing of this formulation revealed a flow rate of 16 seconds confirming the defoamer had no effect on the flow rate.

### Example 8

The following trial included ivermectin and xanthan gum.

The formulation components and methods comprised:
1. 6g Xanthan dissolved in 600ml water.
2. 3.216g Ivermectin dissolved separately in 150ml medium chain mono-/di-glycerides.
3. 5.4g Sodium methyl paraben and 600mg sodium propyl paraben sodium dissolved in 1800ml of water, to this 2.941g sodium selenate and 37.704g cobalt EDTA (14%) were added under stirring.
4. 120g Levamisole HCL was added to the formulation of step 3 under stirring, to this 60g defoamer was added.
5. 68.1g Oxfendazole was dispersed into the formulation of step 4 under stirring.
6. The medium chain mono-/di-glycerides containing ivermectin was sorbed onto 90g silicon dioxide (brand name Aerosil R972) using mortar and pestle.
7. Ivermectin loaded silicon dioxide (brand name Aerosil R972) was dispersed into the formulation from step 5 under stirring.
8. The formulation from Step 1 was added to that from step 7 under stirring.
9. 50ml of purified water was used to rinse the beaker. This was then added to the formulation of step 8.
10. The pH was adjusted to below 4 preferably 3.71 using 30%w/v citric acid solution.
11. The volume was made up to 3000ml using purified water.

The resulting formulation had a reduced flow time of around 12 seconds.

### Example 9

In a further trial abamectin and 0.2% xanthan gum was used.

The formulation components and methods are as example 9 except:
- At step 2, 3g abamectin is dissolved in 150ml medium chain mono-/di-glycerides (instead of ivermectin in example 9.)
- At step 7 the abamectin loaded silicon dioxide (brand name Aerosil R972) is dispersed into the formulation of step 5.
- At step 10 the pH is set at 3.81 instead of 3.71 in example 9.

Testing of this formulation revealed an easily dispersed composition with a reduced flow time of 12 seconds.

### Example 10

In a further trial, a decreased amount of medium chain mono-/di-glycerides and silicon dioxide (brand name Aerosil R972) was used in combination with sodium benzoate and xanthum gum.

The formulation components and methods are as example 9 except:
- At step 1 xanthan gum was dissolved in 150ml of water instead of 600ml in example 9.
- At step 2 ivermectin was dissolved in 15ml of medium chain mono-/di-glycerides instead of 150ml in example 9.
- At step 3 sodium benzoate was added with sodium methyl paraben and sodium propyl paraben and dissolved in 250ml of water instead of 1800ml in example 9.
- At step 9 10ml of purified water was used to rinse the beaker instead of 50ml in example 9.
- At step 11 the volume was made up to 500ml as opposed to 3000ml in example 9.

Testing of this formulation revealed an incompatibility between the sodium benzoate and one of the ingredients in the formula.

### Example 11

In this trial the formulation of example 10 was used to give a reduced quantity of medium chain mono-/di-glycerides, silicon dioxide (brand name Aerosil R972) and xanthan gum. The sodium benzoate was omitted.

The formulation components and method are as example 10 except:
- At step 3 sodium benzoate is omitted.
- At step 10 the pH is adjusted to 3.65 instead of 3.71 in example 10.

Testing this formulation revealed small oil droplets stuck to the inside of the walls of the container indicating not enough silicon dioxide (brand name Aerosil R972) was added to sorb the oil.

### Example 12

In this trial the formulation is the same as example 11 except:
- At step 7 9g of silicon dioxide (Aerosil R972) was added.
- At step 10 the pH is adjusted to 3.71 instead of 3.65 as in example 11.

Testing of this formulation revealed an easily dispersed formulation with a flow time of 14 seconds.

### Example 13

In this trial the formulation is the same as example 12 except:
- At step 2 ivermectin is dissolved in 10ml medium chain mono-/di-glycerides instead of 15ml as in example 14.
- At step 7 6g of silicon dioxide (Aerosil R972) was added

Testing of this formulation revealed an easily dispersed formulation with a flow time of 14 seconds.

### RESULTS

The result of the trials determined example 12 was the best formulation on the basis it stably integrated two pharmaceutical actives in the formulation. The suspension was surfactant free and had a low sedimentation rate resulting in a formulation that can be stored without significant physical change. The formulation also had a reduced flow rate of around 15 seconds through a Ford No. 4 cup to allow for easy and effective administration of the formulation to animals.

In the examples a lipophilic active, ivermectin is firstly solubilised and then sorbed on a hydrophobic grade of silica. The surrounding aqueous solution contains levamisole, an active more stable at low pH. Levamisole salts (Levamisole HCl) are easily soluble in water. This method provides a physical and chemical separation between the sorbed ivermectin/silica and the surrounding solution or suspension containing the second or more active ingredients. It is envisaged that other materials may also be used as the sorbing medium. It will be appreciated that other combinations of actives may be used

### PREFERRED FORMULATIONS

Based on the development work described above, the following preferred formulations were developed. These illustrate the use of the method to make formulations incorporating 2, 3 or 4 actives together with optional mineral additives. These preferred formulations are administered to the animals orally at a rate of 1 ml per 5 kg live animal weight.

### Oral Drench Containing Two Actives and Minerals

### Name: ABAMECTIN - PRAZIQUANTEL DRENCH

| *Materials* | *Amount*/*batch (g)* |
|---|---|
| Abamectin | 500mg |
| Praziquantel | 9.40gms |
| Sodium Selenate | 476mg |
| Cobalt EDTA | 6.284gm |
| Capmul MCM | 15ml |
| Aerosil R972 | 9gms |
| Xanthan gum | 1.250gms |
| Nipagin Sod. | 900mg |
| Nipasol Sod. | 100mg |
| Defoamer | 10gms |
| Citric acid anhydrous | 500mg |
| P. Water Q.S. | 500ml |

### Procedure:

1 Dissolve Xanthan gum in 150 ml of water.
2 Dissolve Abamectin in Capmul MCM.
3 Dissolve Nipagin sodium and Nipasol sodium in water under heating separately. To this add sodium selenate, Cobalt EDTA and defoamer under stirring.
4 Disperse Praziquantel in step 3 under stirring.
5 Adsorb Capmul MCM containing Abamectin solution on Aerosil R972 using mortar and pestle.
6 Disperse Abamectin loaded Aerosil in step 4 under stirring.
7 Add Citric acid to step 6 under stirring.
8 Make the volume with water.
9 Check the pH.

### Oral Drench Containing Two Actives without Minerals

### Name: ABAMECTIN - PRAZIQUANTEL DRENCH

| *Materials* | *Amount*/*batch (g)* |
|---|---|
| Abamectin | 500mg |
| Praziquantel | 9.40gms |
| Capmul MCM | 15.0ml |
| Aerosil R972 | 9.0gms |
| Xanthan gum | 1.250gms |
| Nipagin Sod. | 900mg |
| Nipasol Sod. | 100mg |
| Defoamer | 10gms |
| Citric acid anhydrous | 500mg |
| P. Water Q.S. | 500ml |

### Procedures

1 Dissolve Xanthan gum in 150 ml of water.
2 Dissolve Abamectin in Capmul MCM
3 Dissolve Nipagin sodium and Nipasol sodium in 250 ml of water under heating separately. To this add defoamer under stirring.
4 Disperse Praziquantel in step 3 under stirring
5 Adsorb Capmul MCM containing Abamectin solution on Aerosil R972using mortar and pestle.
6 Disperse Adsorbed abamectin in step 4 under stirring.
7 Add Citric acid to step 6 under stirring.
8 Make the volume with water.
9 Check the pH

### Oral Drench Containing Three Actives and Minerals

### Name: 3 COMPONENT DRENCH

| *Materials* | Amount/batch (g) |
|---|---|
| Levamisole HCl | 20gms |
| Oxfendazole | 11.35gms |
| Abamectin | 500mg |
| Sodium Selenate eq. to selenium | 0.2gms |
| Cobalt EDTA eq. to cobalt | 0.88gms |
| Capmul MCM | 15ml |
| Aerosil R972 | 9gms |
| Xanthan Gum | 1.5gms |
| Nipagin Sod. | 900mg |
| Nipasol Sod. | 100mg |
| Defoamer | 10gm |
| Citric acid anhydrous | 1.4gm |
| P. Water Q.S. | to 500ml |

### PROCEDURES

1 Dissolve Xanthan Gum in 150ml of water
2 Dissolve Abamectin in Capmul MCM separately
3 Dissolve Nipagin sodium and Nipasol sodium in 250ml of water under heat. To this add Sodium Selenate and cobalt EDTA under stirring.
4 Add Levamisole HCl to step 3 under stirring. To this add Defoamer under stirring.
5 Disperse Oxfendazole in step 4 under stirring.
6 Adsorb Capmul MCM containing Abamectin on Aerosil R972 using mortar and pestle.
7 Disperse Abamectin loaded Aerosil in step 5 under stirring.
8 Add step 1 to step 7 under stirring.
9 Add Citric acid anhydrous to step 8 under stirring and check the pH.
10 Make the volume up to 500ml using purified water and stir further 30 minutes.

### Oral Drench Containing Four Actives and Minerals

### Name: 4 COMPONENT DRENCH

| *Materials* | *Amount*/*batch (g)* |
|---|---|
| Levamisole HCl | 40gms |
| Oxfendazole | 22.70gms |
| Abamectin | 1.000gm |
| Praziquantel | 18.80gms |
| Sodium Selenate eq. to selenium | 0.4gms |
| Cobalt EDTA eq. to cobalt | 1.76gms |
| Capmul MCM | 30.00ml |
| Aerosil R972 | 18.00gms |
| Xanthan Gum | 2.500gms |
| Defoamer | 20gms |
| Nipagin Sod. | 1.8gm |
| Nipasol Sod. | 200mg |
| Citric acid anhydrous | 2.8gms |
| P. Water Q.S. | 1000ml |

### Procedures

1 Dissolve Xanthan Gum in 300ml of water
2 Dissolve Abamectin in Capmul MCM separately
3 Dissolve Nipagin sodium and Nipasol sodium in 500ml of water under heat. To this add Sodium selenate and cobalt EDTA under stirring.
4 Add Levamisole HCl to step3 under stirring. To this add Defoamer under stirring.
5 Disperse Oxfendazole in step 4 under stirring. To this add Praziquantel under stirring.
6 Adsorb Capmul MCM containing Abamectin solution on Aerosil R972 using mortar and pestle.
7 Disperse Abamectin loaded Aerosil in step 5 under stirring.
8 Add step 1 to step 7 under stirring.
9 Add citric acid anhydrous to step 8 and check the pH.

Make volume up to 1000ml using purified water and stir further 30 minutes.

The flow time of the 3 and 4 way drench disclosed above and the Triton™ 3-way drench marketed by Nufarm was compared. Flow time was determined using Sheen 406/4; ASTM D1200 cup.

| *S. No.* | *Sample* | *Flow Time (Seconds)* |
|---|---|---|
| 1 | Triton | 33-36 |
| 2 | 3-way drench | 14-17 |
| 3 | 4-way drench | 16-18 |

3-way and 4-way drench formulations are suspension type of dosage forms. Some sediment formed upon standing at room temperature. The sedimented portion was easily redispersed upon shaking. There was no sign of cake formation.

### FIELD TRIALS

The preferred formulations have also been shown to be highly efficacious in field use. The formulations were administered to animals as a drench at a rate of 1 ml per 5 kg animal weight. Tables 1 and 2 below show the arithmetic and geometric mean total worm counts respectively for groups of sheep treated with the 3 and 4 active drenches. Tables 3 and 4 show the efficacies of each of the treatments relative to the controls using arithmetic and geometric means respectively.

Species identification indicated that the following species were present in the untreated control group: *Cooperia* species: 94% C. *curticei,* 4% C. *oncophora,* 2% *C. punctata, Trichostrongylus* species: 100% *T. colubriformis.*

Results demonstrate that both the 3 and 4 active drenches were highly effective against all species present in the trial. Efficacies of >99.9% were achieved against all parasites that were present. Worm numbers in the control animals were high for all species that were present demonstrating that the animals were exposed to a very high parasite challenge.

**Table 1: Arithmetic mean total worm counts for control and treated groups**

| **Treatment** | **Control** | **4 active** | **3 active** |
|---|---|---|---|
| *Ostertagia* (mature) | 8275 | 0 | 0 |
| *Ostertagia* (immature) | 692 | 0 | 0 |
| *T. axei* (mature) | 1350 | 0 | 0 |
| *T. axei* (immature) | 67 | 0 | 0 |
| *H. contortus* (mature) | 5292 | 0 | 0 |
| *H. contortus* (immature) | 2150 | 0 | 0 |
| *Trichostrongylus* spp (mature) | 26983 | 0 | 0 |
| *Trichostrongylus* spp (immature) | 75 | 0 | 0 |
| *Cooperia* (mature) | 2258 | 0 | 0 |
| *Cooperia* (immature) | 58.4 | 0 | 0 |
| *Strongyloides* (mature) | 66.7 | 0 | 0 |
| *Nematodirus* (mature) | 267 | 0 | 0 |
| *Nematodirus* (immature) | 133.4 | 0 | 0 |
| *Oesophagostomum* | 38 | 0 | 0 |
| *Chabertia* | 12 | 0 | 0 |
| *Trichuris* | 17 | 0 | 0 |

**Table 2: Geometric mean total worm counts for control and treated groups**

| **Treatment** | **Control** | **4 active** | **3 active** |
|---|---|---|---|
| *Ostertagia* (mature) | 7660^{a} | 0^{b} | 0^{b} |
| *Ostertagia* (immature) | 140^{a} | 0^{b} | 0^{b} |
| *T. axei* (mature) | 858^{a} | 0^{b} | 0^{b} |
| *T. axei* (immature) | 21^{a} | 0^{b} | 0^{b} |
| *H. contortus* (mature) | 3343^{a} | 0^{b} | 0^{b} |
| *H. contortus* (immature) | 1009^{a} | 0^{b} | 0^{b} |
| *Trichostrongylus* spp (mature) | 20254^{a} | 0^{b} | 0^{b} |
| *Trichostrongylus* spp (immature) | 19.7^{a} | 0^{b} | 0^{b} |
| *Cooperia* (mature) | 1281^{a} | 0^{b} | 0^{b} |
| *Cooperia* (immature) | 11^{a} | 0^{a} | 0^{a} |
| *Strongyloides* (mature) | 18.7^{a} | 0^{b} | 0^{b} |
| *Nematodirus* (mature) | 20.6^{a} | 0^{b} | 0^{b} |
| *Nematodirus* (immature) | 53.8^{a} | 0^{b} | 0^{b} |
| *Oesophagostomum* | 33^{a} | 0^{b} | 0^{b} |
| *Chabertia* | 6^{a} | 0^{b} | 0^{b} |
| *Trichuris* | 15^{a} | 0^{b} | 0^{b} |

| | | | |
|---|---|---|---|
| ^{ab} = means with different superscripts within the same row are statistically different from each other. | | | |

**Table 3: Treatment efficacies based on group arithmetic mean total worm counts.**

| **Treatment** | **4 active** | **3 active** |
|---|---|---|
| *Ostertagia* (mature) | >99.9% | >99.9% |
| *Ostertagia* (immature) | >99.9% | >99.9% |
| *T. axei* (mature) | >99.9% | >99.9% |
| *T. axei* (immature) | >99.9% | >99.9% |
| *H. contortus* (mature) | >99.9% | >99.9% |
| *H. contortus* (immature) | >99.9% | >99.9% |
| *Trichostrongylus* spp (mature) | >99.9% | >99.9% |
| *Trichostrongylus* spp (immature) | >99.9% | >99.9% |
| *Cooperia* (mature) | >99.9% | >99.9% |
| *Cooperia* (immature) | >99.9% | >99.9% |
| *Strongyloides* (mature) | >99.9% | >99.9% |
| *Nematodirus* (mature) | >99.9% | >99.9% |
| *Nematodirus* (immature) | >99.9% | >99.9% |
| *Oesophagostomum* | >99.9% | >99.9% |
| *Chabertia* | >99.9% | >99.9% |
| *Trichuris* | >99.9% | >99.9% |

**Table 4: Treatment efficacies based on group geometric mean total worm counts.**

| **Treatment** | **4 active** | **3 active** |
|---|---|---|
| *Ostertagia* (mature) | >99.9% | >99.9% |
| *Ostertagia* (immature) | >99.9% | >99.9% |
| *T. axei* (mature) | >99.9% | >99.9% |
| *T. axei* (immature) | >99.9% | >99.9% |
| *H. contortus* (mature) | >99.9% | >99.9% |
| *H. contortus* (immature) | >99.9% | >99.9% |
| *Trichostrongylus* spp (mature) | >99.9% | >99.9% |
| *Trichostrongylus* spp (immature) | >99.9% | >99.9% |
| *Cooperia* (mature) | >99.9% | >99.9% |
| *Cooperia* (immature) | >99.9% | >99.9% |
| *Strongyloides* (mature) | >99.9% | >99.9% |
| *Nematodirus* (mature) | >99.9% | >99.9% |
| *Nematodirus* (immature) | >99.9% | >99.9% |
| *Oesophagostomum* | >99.9% | >99.9% |
| *Chabertia* | >99.9% | >99.9% |
| *Trichuris* | >99.9% | >99.9% |

### STABILITY TRIALS

It has also been demonstrated that the formulations made according to this method are stable using acceleraterd testing in elevated temperature conditions. Batch samples were tested for 2, 3, and 4 active drenches.

### Stability results summary of trials

### Stress conditions: 55°C for 2, 4 weeks

| *Batch No.* | *Condition* | *Ivermectin* | % *Recovery* | *Oxfendazole* | % *Recovery* | *Levamisole Hcl* | % *Recovery* |
|---|---|---|---|---|---|---|---|
| Example 1 | 55°C, 4week | 0.083 | 93% | 2.08 | 109% | 3.78 | 97% |
| | 55°C, 2week | 0.088 | 99% | 2.07 | 108% | 3.90 | 101% |
| | 4°C | 0.089 | 100% | 1.91 | 100% | 3.88 | 100% |
| Example 2 | 55°C, 4week | 0.087 | 95% | 2.06 | 99% | 3.88 | 99% |
| | 55°C, 2week | 0.090 | 98% | 2.06 | 100% | 3.99 | 102% |
| | 4°C | 0.092 | 100% | 1.73 | 100% | 3.91 | 100% |
| Example 3 | 55°C, 4week | 0.095 | 97% | 2.33 | 110% | 4.15 | 100% |
| | 55°C, 2week | 0.096 | 98% | 2.24 | 106% | 4.18 | 101% |
| | 4°C | 0.098 | 100% | 2.12 | 100% | 4.13 | 100% |
| Example 4 | 55°C, 4week | 0.079 | 93% | 1.85 | 97% | 3.91 | 102% |
| | 55°C, 2week | 0.079 | 96% | 1.75 | 98% | 3.97 | 104% |
| | 4°C | 0.088 | 100% | 1.91 | 100% | 3.82 | 100% |
| Example 5 | 55°C, 4week | 0.087 | 99% | 2.12 | 104% | 3.80 | 98% |
| | 55°C, 2week | 0.090 | 102% | 2.13 | 104% | 4.05 | 104% |
| | 4°C | 0.088 | 100% | 2.04 | 100% | 3.89 | 100% |
| Example 6 | 55°C, 4week | 0.087 | 84% | 2.46 | 102% | 3.84 | 96% |
| | 55°C, 2week | 0.090 | 87% | 2.42 | 100% | 3.76 | 94% |
| | 4°C | 0.103 | 100% | 2.41 | 100% | 4.00 | 100% |
| Example 7 | 55°C, 4week | 0.096 | 93% | 2.39 | 102% | 3.63 | 95% |
| | 55°C, 2week | 0.099 | 96% | 2.36 | 101% | 3.79 | 99% |
| | 4°C | 0.103 | 100% | 2.34 | 100% | 3.83 | 100% |
| Example 8 | 4°C | 0.107 | 100% | 2.36 | 100% | 4.02 | 100% |
| | 55°C, 2week | 0.103 | 96% | 2.37 | 100% | 3.97 | 99% |
| | 55°C, 4week | 0.099 | 93% | 2.38 | 101% | 3.95 | 98% |
| Example 12 | 4°C | 0.109 | 100% | 2.51 | 100% | 4.23 | 100% |
| | 55°C, 2week | 0.105 | 96% | 2.50 | 100% | 4.17 | 99% |
| | 55°C, 4week | 0.101 | 93% | 2.58 | 103% | 4.25 | 100% |
| Example 13 | 4°C | 0.100 | 100% | 2.39 | 100% | 4.22 | 100% |
| | 55°C, 2week | 0.099 | 99% | 2.43 | 102% | 4.12 | 98% |
| | 55°C, 4week | 0.098 | 98% | 2.43 | 102% | 4.05 | 96% |
| Example 9 | 4°C | 0.103 | 100% | 2.43 | 100% | 4.17 | 100% |
| | 55°C, 2week | 0.101 | 98% | 2.40 | 99% | 4.08 | 98% |
| | 55°C, 4week | 0.096 | 93% | 2.45 | 101% | 3.99 | 96% |

### PREFERRED FORMULATIONS STABILITY RESULTS

### Stability results of 2 actives drench

### Stress conditions: 55°C for 2-4 weeks

| *Batch No.* | *Condition* | *Abamectin* | *% Recovery* | *Praziquantel* | *% Recovery* |
|---|---|---|---|---|---|
| 011202 | 4°C | 0.089 | 100% | 1.97 | 100% |
| (Without Minerals) | 55°C, 2week | 0.089 | 100% | 1.97 | 100% |
| | 55°C, 4week | 0.089 | 100% | 1.94 | 99% |
| 011202 (With Minerals) | 4°C | 0.079 | 100% | 1.83 | 100% |
| | 55°C, 2week | 0.079 | 100% | 1.77 | 97% |
| | 55°C, 4week | 0.079 | 100% | 1.79 | 98% |

### Stability results of 3 actives drench

### Stress conditions: 55°C for 2-4 weeks

| *Batch No.* | *Condition* | *Abamectin* | % *Recovery* | *Oxfendazole* | % *Recovery* | *Levamisole HCl* | % *Recovery* |
|---|---|---|---|---|---|---|---|
| DRE001 /04 | 4°C | 0.108 | 100% | 2.55 | 100% | 4.71 | 100% |
| | 55°C, 2week | 0.102 | 94% | 2.55 | 100% | 4.67 | 100% |
| | 55°C, 4week | 0.099 | 92% | 2.60 | 102% | 3.71 | 100% |

### Stability results of 4 actives drench

### Stress conditions: 55°C for 2-4 weeks

| *Batch No.* | *Condition* | *Ivermectin* | % *Recovery* | *Oxfendazole* | % *Recovery* | *Levamisole HCl* | % *Recovery* | *Praziquantel* | % *Recovery* |
|---|---|---|---|---|---|---|---|---|---|
| 050802 | 4C | 0.124 | 100% | 2.60 | 100% | 4.47 | 100% | 1.99 | 100% |
| | 55C, 2week | 0.133 | 105% | 2.52 | 97% | 4.42 | 99% | 1.96 | 98% |
| | 55C, 4week | 0.139 | 107% | 2.62 | 101% | 4.42 | 99% | 1.96 | 98% |
| 060802 | 4C | 0.111 | 100% | 2.33 | 100% | 4.10 | 100% | 1.87 | 100% |
| | 55C, 2week | 0.100 | 90% | 2.33 | 100% | 4.05 | 99% | 1.86 | 99% |
| | 55C, 4week | 0.100 | 90% | 2.37 | 102% | 4.01 | 98% | 1.88 | 101% |
| 070802 | 4C | 0.114 | 100% | 2.13 | 100% | 4.68 | 100% | 2.09 | 100% |
| | 55C, 2week | 0.116 | 102% | 2.15 | 101% | 4.65 | 99% | 2.10 | 100% |
| | 55C, 4week | 0.113 | 99% | 2.14 | 100% | 4.63 | 99% | 2.09 | 100% |
| 080802 | 4C | 0.099 | 100% | 2.08 | 100% | 3.95 | 100% | 1.84 | 100% |
| | 55C, 2week | 0.098 | 99% | 2.07 | 100% | 3.98 | 101% | 1.63 | 95% |
| | 55C, 4week | 0.098 | 99% | 2.06 | 99% | 4.01 | 102% | 1.59 | 93% |
| 090802 | 4C | 0.104 | 100% | 2.46 | 100% | 3.21 | 100% | 2.05 | 100% |
| | 55C, 2week | 0.104 | 100% | 2.42 | 98% | 3.19 | 99% | 2.06 | 100% |
| | 55 C, 4week | 0.099 | 95% | 2.34 | 95% | 3.20 | 100% | 2.00 | 98% |
| 100802 | 4C | 0.082 | 100% | 3.85 | 100% | 4.07 | 100% | 1.85 | 100% |
| | 55C, 2week | 0.081 | 99% | 3.85 | 100% | 4.04 | 99% | 1.80 | 97% |
| | 55C, 4week | 0.083 | 101% | 3.95 | 103% | 4.04 | 99% | 1.85 | 100% |
| 110802 | 4C | 0.086 | 100% | 2.52 | 100% | 4.39 | 100% | 2.01 | 100% |
| | 55C, 2week | 0.086 | 100% | 2.53 | 100% | 4.32 | 98% | 2.01 | 100% |
| | 55C, 4week | 0.088 | 102% | 2.59 | 103% | 4.30 | 98% | 2.05 | 102% |

As can be seen from the above trials the methods of the present description and the formulations made from those methods, allow the stable integration of multiple actives within a single formulation. The solubilisation of the active in a solvent and the subsequent adsorption onto a sorbing medium provide effective protection for the active from any adverse conditions within the liquid in which the active-loaded sorbing medium is dispersed. As a result the liquid can be formulated to suit the requirements of the actives that may include therein. As a result the actives may be stably integrated within the formulation. This allows the co-administration of the actives and the resultant benefits in terms of cost savings and convenience to be enjoyed.

## Claims

1. A method of manufacturing a pharmaceutical formulation comprising the steps:
a) dissolving an active in a solvent, wherein the active is a lipophilic active and wherein the lipophilic active is selected from the group consisting of avermectins and the milbemycins;
b) sorbing the solvent containing active composition on to a sorbing medium wherein the sorbing medium is hydrophobic fumed silica; and then
c) dispersing the sorbing medium loaded with the solvent composition in a liquid comprising one or more actives dissolved or suspended therein wherein the active is a vitamin, mineral, anthelmintics or antigen and wherein the liquid is an aqueous vehicle.

2. The method as claimed in claim 1, wherein the solvent is selected from oils and organic solvents.

3. The method as claimed in claim 2, wherein the solvent is selected from medium chain mono- /di-glycerides in the range of 0.1-10%.

4. The method as claimed in claim 2, wherein the solvent is selected from medium chain mono- /di-glycerides in the range of between 2-4%.

5. The method as claimed in any one of claims 1 to 4, wherein the minerals are selected from selenium salts, cobalt salts, copper salts, zinc salts, iodine salts and their chelates.

6. The method as claimed in claim 5 wherein the minerals are selected from sodium selenate and cobalt EDTA.

7. The method as claimed in any one of claims 1 to 6 wherein the anthelmintics are selected from the group including a levamisole salt, albendazole, oxfendazole, fenbendazole, mebendazole, acylated quinoline, praziquantel, benzenesulphonamide, clorsulon and closantel.

8. The method as claimed in any previous claim, wherein the liquid comprises further excipients.

9. A pharmaceutical formulation capable of dilution and capable (with or without dilution) of being administered to an animal, said formulation comprising:
a) an active dissolved in a suitable solvent to form a solution and sorbed on to a sorbing medium, wherein the active is a lipophilic active and wherein the lipophilic active is selected from the group consisting of avermectins and the milbemycins, and wherein the sorbing medium is hydrophobic fumed silica; and
b) a liquid diluent wherein the liquid diluent is an aqueous vehicle comprising one or more actives dissolved or suspended therein wherein the active is a vitamin, mineral, anthelmintics or antigen.

10. The formulation as claimed in claim 9, wherein the solvent is selected from oils and organic solvents.

11. The formulation as claimed in claim 10, wherein the solvent is medium chain mono-/di- glycerides.

12. The formulation as claimed in any one of claims 9 to 11 wherein the minerals are selected from selenium salts, cobalt salts, copper salts, zinc salts, iodine salts and their chelates.

13. The formulation as claimed in claim 12 wherein the minerals are selected from sodium selenate and cobalt EDTA.

14. The formulation as claimed in any one of claims 9 to 13 wherein the anthelmintics are selected from the group including a levamisole salt, albendazole, oxfendazole, fenbendazole, mebendazole, acylated quinoline, praziquantel, benzenesulphonamide, clorsulon and closantel.

15. The formulation as claimed in any of claims 9 to 14, which additionally includes preservatives, suspending agents, buffering agents, and antifoaming agents.

16. Use of a formulation as described in any one of claims 9 to 15 in the manufacture of a medicament formulated for administration to an animal.

17. A formulation as described in any one of claims 9 to 15 for use in a medicament formulated for administration to an animal.

## Patentansprüche

1. Verfahren zur Herstellung einer pharmazeutischen Formulierung, umfassend die Schritte:
a) Lösen eines Wirkstoffs in einem Lösungsmittel, wobei der Wirkstoff ein lipophiler Wirkstoff ist, und wobei der lipophile Wirkstoff ausgewählt ist aus der Gruppe bestehend aus Avermectinen und den Milbemycinen;
b) Absorbieren der Wirkstoffzusammensetzung, die das Lösungsmittel enthält, an ein Sorptionsmedium, wobei das Sorptionsmedium hydrophobe pyrogene Kieselsäure ist; und dann
c) Dispergieren des mit der Lösungsmittelzusammensetzung beladenen Sorptionsmediums in einer Flüssigkeit, die einen oder mehrere darin gelöste oder suspendierte Wirkstoff(e) umfasst, wobei der Wirkstoff ein Vitamin, Mineralstoff, Anthelminthika, oder Antigen ist, und wobei die Flüssigkeit ein wässriges Vehikel ist.

2. Verfahren wie in Anspruch 1 beansprucht, wobei das Lösungsmittel aus Ölen und organischen Lösungsmitteln ausgewählt ist.

3. Verfahren wie in Anspruch 2 beansprucht, wobei das Lösungsmittel aus mittelkettigen Mono-/Diglyceriden in einem Bereich von 0,1-10% ausgewählt ist.

4. Verfahren wie in Anspruch 2 beansprucht, wobei das Lösungsmittel aus mittelkettigen Mono-/Diglyceriden in einem Bereich von 2-4% ausgewählt ist.

5. Verfahren wie in einem der Ansprüche 1 bis 4 beansprucht, wobei die Mineralstoffe aus Seleniumsalzen, Kobaltsalzen, Kupfersalzen, Zinksalzen, Jodsalzen und deren Chelaten ausgewählt sind.

6. Verfahren wie in Anspruch 5 beansprucht, wobei die Mineralstoffe aus Natriumselenat und Kobalt-EDTA ausgewählt sind.

7. Verfahren wie in einem der Ansprüche 1 bis 6 beansprucht, wobei die Anthelminthika ausgewählt sind aus der Gruppe, umfassend ein Levamisol-Salz, Albendazol, Oxfendazol, Fenbendazol, Mebendazol, acyliertes Chinolin, Praziquantel, Benzolsulfonamid, Clorsulon und Closantel.

8. Verfahren wie in einem der vorherigen Ansprüche beansprucht, wobei die Flüssigkeit weitere Hilfsstoffe umfasst.

9. Pharmazeutische Formulierung, die zur Verdünnung geeignet ist und (verdünnt oder unverdünnt) dazu geeignet ist einem Tier verabreicht zu werden, wobei die Formulierung umfasst:
a) einen Wirkstoff, der in einem geeigneten Lösungsmittel gelöst ist, um eine Lösung zu erzeugen, und der an ein Sorptionsmedium absorbiert ist, wobei der Wirkstoff ein lipophiler Wirkstoff ist, und wobei der lipophile Wirkstoff ausgewählt ist aus der Gruppe bestehend aus Avermectinen und den Milbemycinen, und wobei das Sorptionsmedium hydrophobe pyrogene Kieselsäure ist; und
b) ein flüssiges Verdünnungsmittel, wobei das flüssige Verdünnungsmittel ein wässriges Vehikel ist, das einen oder mehrere darin gelöste oder suspendierte Wirkstoff(e) umfasst, wobei der Wirkstoff ein Vitamin, Mineralstoff, Anthelminthika, oder Antigen ist.

10. Formulierung wie in Anspruch 9 beansprucht, wobei das Lösungsmittel aus Ölen und organischen Lösungsmitteln ausgewählt ist.

11. Formulierung wie in Anspruch 10 beansprucht, wobei das Lösungsmittel mittelkettige Mono-/Diglyceride sind.

12. Formulierung wie in einem der Ansprüche 9 bis 11 beansprucht, wobei die Mineralstoffe aus Seleniumsalzen, Kobaltsalzen, Kupfersalzen, Zinksalzen, Jodsalzen und deren Chelaten ausgewählt sind.

13. Formulierung wie in Anspruch 12 beansprucht, wobei die Mineralstoffe aus Natriumselenat und Kobalt-EDTA ausgewählt sind.

14. Formulierung wie in einem der Ansprüche 9 bis 13 beansprucht, wobei die Anthelminthika ausgewählt sind aus der Gruppe, umfassend ein Levamisol-Salz, Albendazol, Oxfendazol, Fenbendazol, Mebendazol, acyliertes Chinolin, Praziquantel, Benzolsulfonamid, Clorsulon und Closantel.

15. Formulierung wie in einem der Ansprüche 9 bis 14 beansprucht, die zusätzlich Konservierungsmittel, suspendierende Agentien, Puffersubstanzen und Entschäumer umfasst.

16. Verwendung einer wie in einem der Ansprüche 9 bis 15 beschriebenen Formulierungen zur Herstellung eines Medikaments, das für die Verabreichung an ein Tier formuliert ist.

17. Formulierung wie in einem der Ansprüche 9 bis 15 beschrieben zur Verwendung in einem Medikament, das für die Verabreichung an ein Tier formuliert ist.

## Revendications

1. Procédé de préparation d'une formulation pharmaceutique comprenant les étapes de :
a) dissolution d'un principe actif dans un solvant, dans laquelle le principe actif est un principe actif lipophile et dans laquelle le principe actif lipophile est choisi dans le groupe constitué par des avermectines et les milbémycines ;
b) sorption de la composition active contenant le solvant sur un milieu de sorption, dans laquelle le milieu de sorption est une silice pyrogénée hydrophobe ; et ensuite
c) dispersion du milieu de sorption chargé de la composition de solvant dans un liquide comprenant un ou plusieurs principes actifs dissous ou en suspension en son sein, dans laquelle le principe actif est une vitamine, un minéral, des composés anthelmintiques ou un antigène et dans laquelle le liquide est un vecteur aqueux.

2. Procédé tel que revendiqué à la revendication 1, dans lequel le solvant est choisi parmi des huiles et des solvants organiques.

3. Procédé tel que revendiqué à la revendication 2, dans lequel le solvant est choisi parmi des mono-/di-glycérides à chaîne moyenne dans une plage allant de 0,1 à 10 %.

4. Procédé tel que revendiqué à la revendication 2, dans lequel le solvant est choisi parmi des mono-/di-glycérides à chaîne moyenne dans une plage comprise entre 2 et 4%.

5. Procédé tel que revendiqué dans l'une quelconque des revendications 1 à 4, dans lequel les minéraux sont choisis parmi des sels de sélénium, des sels de cobalt, des sels de cuivre, des sels de zinc, des sels d'iode et leurs chélates.

6. Procédé tel que revendiqué dans la revendication 5, dans lequel les minéraux sont choisis parmi le séléniate de sodium et l'EDTA de cobalt.

7. Procédé tel que revendiqué à l'une quelconque des revendications 1 à 6, dans lequel les composés anthelmintiques sont choisis dans le groupe comprenant un sel de lévamisole, de l'albendazole, de l'oxfendazole, du fenbendazole, du mebendazole, de la quinoléine acylée, du praziquantel, du benzène sulfonamide, du clorsulon et du closantel.

8. Procédé tel que revendiqué à l'une quelconque des revendications précédentes, dans lequel le liquide comprend d'autres excipients.

9. Formulation pharmaceutique apte à être diluée et à être administrée (après avoir été diluée ou non) à un animal, ladite formulation comprenant :
a) un principe actif dissous dans un solvant approprié pour former une solution et sorbé sur un milieu de sorption, dans laquelle le principe actif est un principe actif lipophile et dans laquelle le principe actif lipophile est choisi dans le groupe constitué par des avermectines et les milbémycines, et dans laquelle le milieu de sorption est une silice pyrogénée hydrophobe ; et
b) un diluant liquide, dans laquelle le diluant liquide est un vecteur aqueux comprenant un ou plusieurs principes actifs dissous ou en suspension en son sein dans laquelle le principe actif est une vitamine, un minéral, des composés anthelmintiques ou un antigène.

10. Formulation telle que revendiquée à la revendication 9, dans laquelle le solvant est choisi parmi des huiles et des solvants organiques.

11. Formulation telle que revendiquée à la revendication 10, dans laquelle le solvant consiste en des mono-/di-glycérides à chaîne moyenne.

12. Formulation telle que revendiquée à l'une quelconque des revendications 9 à 11, dans laquelle les minéraux sont choisis parmi des sels de sélénium, des sels de cobalt, des sels de cuivre, des sels de zinc, des sels d'iode et leurs chélates.

13. Formulation telle que revendiquée à la revendication 12, dans laquelle les minéraux sont choisis parmi le séléniate de sodium et l'EDTA de cobalt.

14. Formulation telle que revendiquée à l'une quelconque des revendications 9 à 13, dans laquelle les composés anthelmintiques sont choisis dans le groupe comprenant un sel de lévamisole, de l'albendazole, de l'oxfendazole, du fenbendazole, du mebendazole, de la quinoléine acylée, du praziquantel, du benzène sulfonamide, du clorsulon et du closantel.

15. Formulation telle que revendiquée à l'une quelconque des revendications 9 à 14, qui comprend en outre des agents de conservation, des agents de mise en suspension, des agents tampons, et des agents anti-mousse.

16. Utilisation d'une formulation telle que décrite à l'une quelconque des revendications 9 à 15, dans la fabrication d'un médicament formulé pour être administré à un animal.

17. Formulation telle que décrite à l'une quelconque des revendications 9 à 15, pour utilisation dans un médicament formulé pour être administré à un animal.
